# FASCICULE DE BREVET EUROPEEN

(11) **EP 4 132 439 B1**
(45) Date de publication et mention de la délivrance du brevet: **05.06.2024**
(21) Numéro de dépôt: 21723333.7
(22) Date de dépôt: 06.04.2021
(51) Int. Cl.: A61F 9/008

(54) **APPAREIL DE CHIRURGIE OPHTALMIQUE**
AUGENCHIRURGIEGERÄT
OPHTHALMIC SURGERY APPARATUS

(30) Priorité: 07.04.2020 FR 2003451
(43) Date de publication de la demande: 15.02.2023
(73) Titulaire: UNIVERSITE DE BORDEAUX, 33000 Bordeaux (FR); Centre National de la Recherche Scientifique (CNRS), 75016 Paris (FR); Centre Hospitalier Universitaire de Bordeaux, 33400 Talence (FR); Centre Technologique Alphanov, 33400 Talence (FR)
(72) Inventeur: LOPEZ, John, 33170 Gradignan (FR); IRIBARREN, Donetsi, 33270 Floirac (FR); CHASSAGNE, Bruno, 33600 Pessac (FR); TOUBOUL, David, 33000 Bordeaux (FR)
(74) Mandataire: Plasseraud IP
(86) Numéro de dépôt international: PCT/FR2021/050599
(87) Numéro de publication internationale: WO 2021/205111

(56) Documents cités:
- EP-A2- 1 981 426
- WO-A1-2019/145484
- CA-A1- 2 769 099
- US-A1- 2014 316 389
- US-A1- 2015 374 549
- US-A1- 2017 266 048
- US-A1- 2018 064 577

## Description

### Domaine technique

L'invention relève du domaine des appareils de chirurgie oculaire. Plus précisément, l'invention concerne un appareil de chirurgie ophtalmologique pour assister le chirurgien à la réalisation des greffes de cornées et la chirurgie réfractive oculaire.

L'invention concerne un appareil de chirurgie ophtalmologique, au moyen d'un laser picoseconde.

Dans la présente demande de brevet, par laser picoseconde, on entend une source lumineuse, apte à émettre un faisceau laser sous forme d'impulsions ultra-courtes, dont la durée d'impulsion est comprise entre quelques centaines de femtosecondes et quelques dizaines de picosecondes.

### Technique antérieure

La cornée fait partie de la tunique périphérique de l'œil. C'est un milieu diélectrique transparent comme le verre, taillé en coupole à faces presque parallèles formant un dioptre asphérique biconvexe. Son diamètre moyen est d'environ 12 mm et son épaisseur moyenne centrale est d'environ 540 microns. Elle présente un rayon de courbure moyen de l'ordre 7,8 mm. Elle permet de faire converger les rayons lumineux incidents vers la rétine pour y former une image.

La cornée est composée de cinq couches distinctes, de la plus externe à la plus interne :
- l'épithélium qui est composé de cinq à sept couches de cellules stratifiées, a renouvellement permanent ;
- la couche de Bowman qui est acellulaire, non renouvelable ;
- le stroma qui constitue 90% de l'épaisseur de la cornée. Elle est composée de lamelles de collagènes et de cellules baignant dans un gel matriciel, lentement renouvelé ;
- la membrane de Descemet qui supporte l'endothélium ;
- l'endothélium qui est en contact avec l'humeur aqueuse, est la couche la plus profonde constituée d'une couche unistratifiée de cellules qui ne renouvellent pas.

Sous l'effet d'un accident de la vie ou d'une pathologie spécifique, la cornée peut s'opacifier partiellement ou totalement, dégradant ainsi la vision. La greffe de cornée constitue alors le seul traitement efficace pour restituer la fonction de la cornée malade. Elle permet en outre de rétablir une bonne acuité visuelle et de supprimer les douleurs provoquées par les lésions cornéennes. La kératoplastie, ou greffe de cornée consiste généralement à extraire une partie ou la totalité d'une cornée pathologique, et à la remplacer par une cornée saine, provenant d'un donneur. Actuellement, cette opération est le plus souvent réalisée de manière manuelle, à l'aide de trépans et/ou de microkératomes mécaniques, par le chirurgien.

Il est également possible de faire appel à la chirurgie réfractive pour compenser les défauts de la vision, dits amétropies, traduisant d'une défaillance de focalisation des rayons lumineux sur le plan de la rétine à travers la rétine. Les amétropies opérables sont la myopie, l'hypermétropie, l'astigmatisme et la presbytie. Les lasers sont actuellement utilisés pour assister le chirurgien à venir traiter la cornée saine pour corriger les défauts de visions.

On peut citer ici l'exemple de la technique du LASIK qui consiste à découper superficiellement un volet de cornée d'une épaisseur de 90 à 120 microns à l'aide d'un microkératome mécanique (lame) ou d'un laser femtoseconde pour ensuite soulever ce volet le temps de remodeler la cornée par dessous. Le laser Excimer type ArF avec un rayonnement ultraviolet à 193nm et une durée d'impulsion de 10 à 25ns permet un remodelage très précis (environ 0,25 microns de photoablation par impulsion). Cette procédure est la plus pratiquée actuellement pour traiter la myopie.

On mentionnera également la spécificité des techniques de SMILE ou RELEX qui consistent à découper une lenticule de cornée dans l'épaisseur de la cornée, à l'aide d'un laser femtoseconde puis à l'extraire manuellement par une incision périphérique, évitant ainsi le recours au laser Excimer. Cette procédure est en pleine expansion.

Actuellement, les appareils de chirurgie ophtalmologique visant des opérations de découpe de cornée sont essentiellement équipés de lasers femtoseconde. Un laser femtoseconde est un laser qui délivre des impulsions de durée comprise entre 1 et quelques centaines de femtosecondes. La brièveté des impulsions permet de concentrer l'énergie laser sur une fenêtre temporelle extrêmement courte, de manière à atteindre des intensités très élevées, de l'ordre de 10¹² à 10¹⁴ W/cm2, une fois focalisée sur la cible. Les impulsions lasers sont focalisées dans l'épaisseur de la cornée selon une trajectoire qui suit une ligne droite ou rectiligne de manière à réaliser une surface de découpe. L'utilisation d'un laser femtoseconde permet de minimiser l'énergie déposée par impulsion afin d'éviter des effets thermiques tout ayant une densité d'énergie suffisante pour provoquer l'apparition d'une bulle de cavitation induite par la photodisruption.

Un laser femtoseconde nécessite généralement l'utilisation de composants optiques et d'un circuit laser complexe. En particulier, pour la plupart des dispositifs disponibles il est nécessaire d'utiliser un système d'amplification complexe très sensible à son environnement de manière à pouvoir amplifier le rayonnement laser sans en endommager les composants internes. Le coût des appareils de chirurgie ophtalmologiques assistés par laser femtoseconde reste en l'occurrence relativement élevé à l'achat et pour la maintenance.

La solution du transport par fibre optique existe expérimentalement mais reste très limitée et contraignante pour des impulsions laser femtosecondes amplifiées.

Les appareils équipés de lasers femtosecondes sont généralement très encombrants et présentent un poids élevé. De ce fait, ils sont généralement peu mobiles et nécessitent souvent l'usage d'une salle dédiée, essentiellement dévouée à la chirurgie réfractive, donc peu accessibles pour les greffes de cornées ou la chirurgie du cristallin.

Des lasers femtosecondes n'utilisant pas d'amplification des impulsions existent, mais la faible énergie par impulsion doit alors être compensée par un objectif de focalisation à grande ouverture numérique. Ce type d'objectif permet d'obtenir un spot laser à l'échelle du micron sur un champ de travail très fable, de l'ordre de 1mm. Il est donc nécessaire d'utiliser une platine motorisée pour déplacer le faisceau laser afin de couvrir la surface à découper (proche 10 mm de diamètre), ce qui limite la précision des découpes malgré un spot de diamètre de quelques microns. A titre d'exemple, dans le cas de l'opération LASIK, la qualité de découpe du plan frontal (lit de la découpe) est essentielle. Par qualité on entend la facilité de clivage et la rugosité des plans de découpe. Dans le cadre de la découpe de cornée, il est ainsi essentiel de minimiser cette rugosité en utilisant des plans de découpe les plus fins possibles. Il est également important de garantir la précision de la découpe afin de limiter la diffraction et garantir une qualité de vision optimale. Dans ce contexte, il convient de noter qu'un spot laser de diamètre 5 microns provoquera une photo-disruption sur 15 à 25 microns avec un laser femtoseconde amplifié, et moins de 5 à 10 microns avec un laser non amplifié. Le geste opératoire recherchant une précision micrométrique, les réglages de la physique des impulsions et du système optiques sont des éléments cruciaux pour atteindre l'efficacité chirurgicale attendue. Également, la qualité de découpe des berges est un élément important dans la greffe de la cornée. Or dans les appareils actuels de chirurgie ophtalmologique, il est difficile de combiner une grande ouverture numérique et un grand champ de travail afin de couvrir toutes les trajectoires de découpes dans des conditions d'efficacité optimisée.

Les appareils actuels pour réaliser les opérations de chirurgie réfractive nécessitent également un chirurgien expérimenté pour obtenir la bonne synchronisation entre l'opérateur et la machine tant les gestes requis sont complexes et précis. Une formation relativement longue des praticiens est ainsi nécessaire.

EP1981426A2, US2018/064577A1, et WO2019/145484A1 divulguent des appareils de chirurgie ophtalmologique pour réaliser une découpe dans un tissu biologique oculaire tel qu'une cornée ou un cristallin. 11

Un objectif de la présente invention est donc de proposer un appareil de chirurgie ophtalmologique, particulièrement adapté à la kératoplastie et à la chirurgie réfractive de la cornée ultra-résolutive. Il sera optimisé en termes d'ergonomie, de compacité, robustesse, légèreté et mobilité par rapport aux dispositifs actuels. Enfin, des efforts portant sur une meilleure automatisation de l'outil permettront de garantir plus de sécurité, d'efficacité et de versatilité pour les procédures.

Un autre objectif de la présente invention est de proposer un design optique et des paramètres laser spécifiquement choisis pour obtenir un appareil de chirurgie ophtalmologique apte à réaliser les découpes tissulaires les plus précises possibles, quelles que soient les contraintes géométriques des trajectoires imposées.

### Exposé de l'invention

Afin de remédier aux inconvénients précités de l'état de la technique, la présente invention propose un appareil de chirurgie ophtalmologique pour réaliser une découpe dans un tissu biologique oculaire, tel qu'une cornée ou un cristallin, comprenant :
- une source laser adaptée pour délivrer un faisceau d'impulsions laser ;
- un système optique de focalisation pour focaliser le faisceau d'impulsions laser en un point focal dans le tissu biologique oculaire;
- un système optique de déplacement du faisceau d'impulsion laser configuré pour déplacer le point focal suivant une trajectoire tridimensionnelle prédéterminée;
- une unité de commande configurée pour piloter la source laser et le système optique de déplacement du faisceau d'impulsions laser de sorte que les paramètres du faisceau d'impulsions laser et les paramètres du système optique de déplacement du faisceau d'impulsion laser soient ajustés en fonction de la position du point focal dans la trajectoire pendant la découpe,
- les paramètres étant la durée d'impulsion du faisceau laser, l'énergie par impulsion, la cadence d'impulsion du faisceau laser et la vitesse de balayage du faisceau laser,
- ladite unité de commande étant configurée pour piloter de manière synchronisée la source laser et le système optique de déplacement de sorte que la durée d'impulsion de la source laser varie en fonction de la position du point focal dans la trajectoire tridimensionnelle prédéterminée.

Avantageusement, ladite unité de commande est apte à piloter la source laser de sorte que la durée d'impulsion du faisceau laser soit comprise entre 350 femtosecondes et 3 picosecondes, de préférence comprise entre 700 femtosecondes et 1,5 picosecondes.

Selon une autre forme de réalisation avantageuse, ladite unité de commande est apte à piloter la source laser de sorte que l'énergie par impulsion soit comprise entre 0,1 µJ et 20 µJ et la cadence comprise entre 50kHz et 2 MHz, de préférence entre 50 kHz et 1 MHz.

Avantageusement, ladite unité de commande est apte à piloter le système de déplacement du faisceau d'impulsion laser de sorte que la vitesse de balayage soit comprise entre 0,1 m/s et 10 m/s.

Les caractéristiques exposées dans les paragraphes suivants peuvent, optionnellement, être mises en oeuvre. Elles peuvent être mises en oeuvre indépendamment les unes des autres ou en combinaison les unes avec les autres :
- le système de déplacement du faisceau d'impulsion laser comporte un premier scanner adapté pour recevoir le faisceau d'impulsions laser incident et configuré de manière à induire un déplacement du faisceau d'impulsion laser suivant un axe Z et un second scanner adapté pour recevoir le faisceau d'impulsion laser incident et configuré de manière à induire un déplacement du faisceau dans un plan (XY) ;
- le système optique de focalisation est disposé entre le système de déplacement optique et le tissu biologique et configuré pour former dans le tissu biologique un point focal de diamètre inférieur à 8,5 µm, de préférence inférieure à 6 µm sur un champ de diamètre compris entre 9 mm et 12 mm ;
- le système optique de focalisation est constitué par une combinaison optique télécentrique comportant une ouverture numérique comprise entre 0,13 et 0,22, de préférence entre 0,20 et 0,22.

Selon une forme de réalisation particulièrement avantageuse, l'appareil de chirurgie ophtalmologique comprend en outre au moins une caméra configurée pour visualiser la zone de découpe.

De préférence, ladite au moins une caméra est agencée entre le système optique de focalisation et le tissu biologique de sorte que le faisceau d'imagerie incident soit incliné d'un angle compris entre 30° et 50°, de préférence compris entre 45° et 47, par rapport à un axe optique de symétrie du système optique de focalisation.

Selon une autre forme de réalisation particulièrement avantageuse, l'appareil de chirurgie ophtalmologique comprend en outre une caméra de centrage configurée pour le centrage de l'axe optique de symétrie du système de focalisation par rapport au tissu biologique.

De préférence, la source laser utilisée émet des impulsions laser à une longueur d'onde comprise entre 1020 nm et 1600 nm, de préférence entre 1030 nm et 1090 nm.

Selon un mode de réalisation particulier, la source laser étant formée de deux parties distinctes, la première partie comportant une cavité laser oscillatrice et un étireur et la seconde partie comportant une cavité laser amplificatrice, un compresseur et un module acousto-optique, ledit appareil comprend d'une part un module de découpe intégrant le système optique de focalisation, le système optique de déplacement du faisceau d'impulsions laser et la seconde partie de la source laser, et d'autre part, une liaison à fibre optique pour transmettre le faisceau laser généré par la première partie de la source laser vers le module de découpe.

Avantageusement, l'appareil de chirurgie ophtalmologique comprend en outre un dispositif interface d'aplanation comprenant une lame à faces planes et parallèles et/ou une lame plan-concave.

L'invention propose également un équipement de chirurgie ophtalmologique pour réaliser une découpe dans un tissu biologique oculaire, tel qu'une cornée ou un cristallin, comprenant un bras articulé auto-équilibré suivant trois axes X, Y et Z, et un appareil de chirurgie ophtalmologique tel que défini ci-dessus, ledit bras comprenant une extrémité reliée à une baie électrotechnique mobile et une extrémité adaptée pour être couplée au module de découpe.

### Brève description des dessins

D'autres caractéristiques, détails et avantages de l'invention apparaîtront à la lecture de la description détaillée ci-après, et à l'analyse des dessins annexés, sur lesquels :
**Fig. 1**

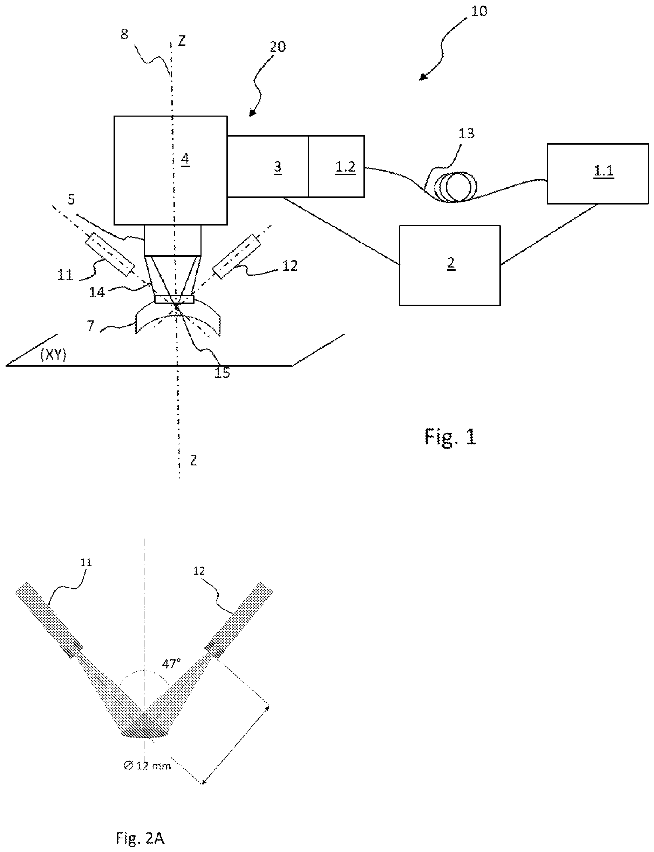

   [Fig. 1] La figure 1 représente schématiquement une vue générale d'un appareil de chirurgie ophtalmologique selon un mode de réalisation de l'invention pour la découpe dans un tissu biologique, tel que la cornée ;
**Fig. 2A**
   [Fig. 2A] La figure 2A représente schématiquement une vue de face de l'agencement des deux caméras de vision latérales ;
**Fig. 2B**

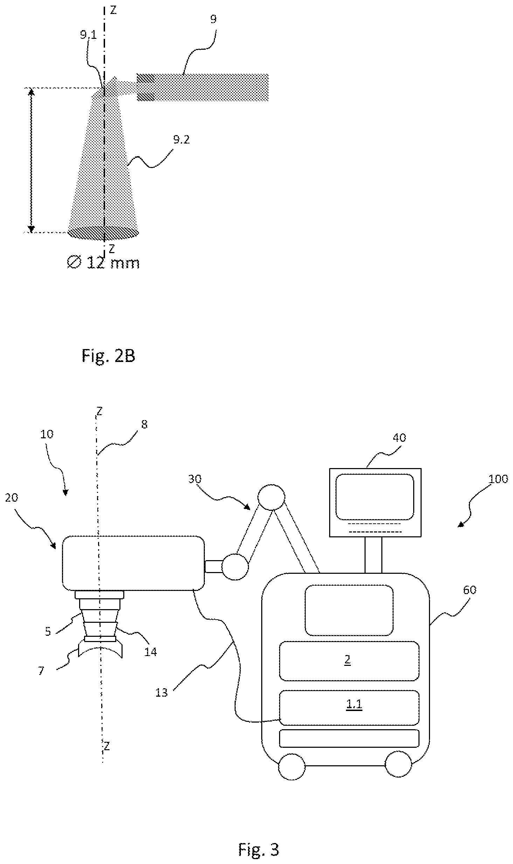

   [Fig. 2B] La figure 2B représente schématiquement une vue de face de l'agencement de la caméra de centrage ;
**Fig. 3**
   [Fig. 3] La figure 3 représente une vue globale d'un équipement de chirurgie ophtalmologique comprenant un bras de robot et un module de découpe intégrant une partie des éléments de l'appareil de chirurgie ophtalmologique de la figure 1 ;
**Fig. 4**

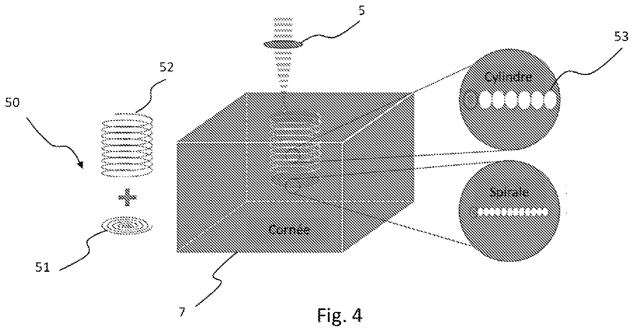

   [Fig. 4] La figure 4 représente la formation de bulles de cavitation sous l'effet des impacts d'un faisceau laser dans la cornée en fonction de la durée d'impulsion pour une trajectoire constituée d'une spirale et d'une hélice ;
**Fig. 5**

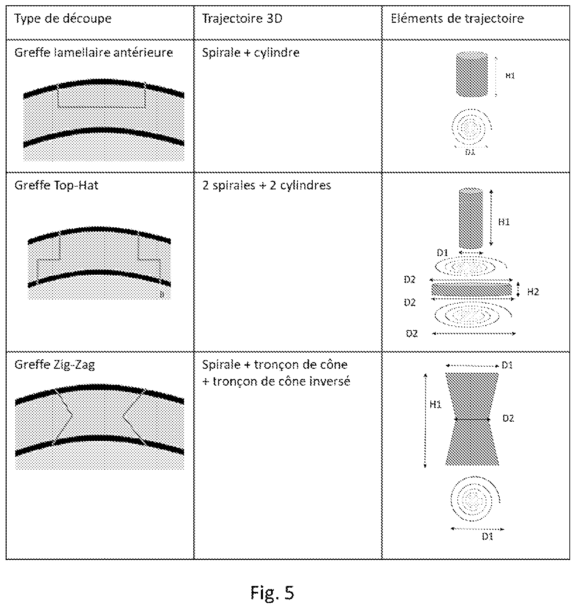

   [Fig. 5] La figure 5 représente schématique trois exemples de trajectoires associées à trois types de découpe ;
**Fig. 6**

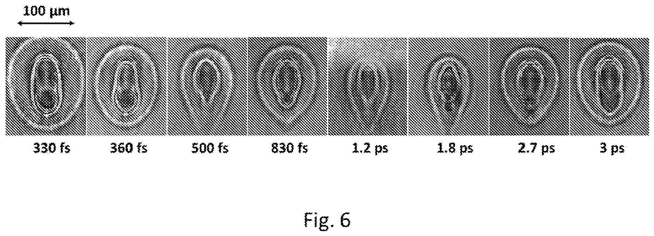

   [Fig. 6] La figure 6 représente une série d'images de vues latérales d'une zone d'irradiation obtenue dans un verre pour des durées d'impulsion comprises entre 330 fs et 3 ps, avec la longueur d'onde fixée à 1030 nm, la cadence fixée à 500 kHz et l'énergie par impulsion fixée à 2 µJ.

Pour plus de clarté, les éléments ou similaires sont repérés par des signes de référence identiques sur l'ensemble des figures.

On entend, dans le cadre de la présente demande, par « laser picoseconde », un laser qui délivre des impulsions de durée comprise entre quelques centaines de femtosecondes et quelques dizaines de picosecondes.

On entend, dans le cadre de la présente demande, par « densité d'énergie », une quantité d'énergie par unité de volume.

On entend, dans le cadre de la présente demande, par « cadence », le nombre d'impulsions par seconde. Augmenter la cadence permet de diminuer le temps de traitement, cependant des phénomènes indésirables peuvent apparaître à haute cadence. En effet, lorsque le délai entre deux impulsions successives est plus court que le temps de relaxation thermique du tissu biologique cible, il y a accumulation thermique et la température du matériau augmente progressivement. Cette charge thermique induit une zone affectée thermiquement et/ou une zone de modification physico-chimique autour de la zone traitée. Par conséquent, l'augmentation de la cadence s'accompagne nécessairement d'une augmentation de la vitesse de balayage de manière à conserver la formation de spots contigus dans le plan focal afin de maintenir la qualité de découpe.

L'ouverture numérique (O.N) = n*sinθ, où le n est l'indice de réfraction du milieu et θ le demi-angle d'incidence du faisceau laser. Une grande ouverture numérique permet d'obtenir un spot laser avec un faible diamètre mais avec une profondeur de champ plus faible. En contrepartie, une grande ouverture numérique génère un champ de travail plus faible. Dans le cadre de la présente demande, il est donc essentiel de pouvoir combiner une ouverture numérique suffisamment grande pour générer un spot de diamètre acceptable dans le cadre de la découpe de cornée et un champ de travail suffisant de manière à pouvoir inclure toute la surface de la pupille.

Le seuil de claquage optique ou de photodisruption correspond au seuil d'énergie par impulsion à partir duquel il y a une formation de bulles de cavitation dans un tissu biologique. La photodisruption est un mécanisme de transformation du tissu de cornée lorsqu'il reçoit un faisceau laser à impulsions ultra-courtes ou ultrabrèves. Le tissu de cornée de la zone d'impact se transforme en plasma qui se dilate et une cavitation de bulle se forme. L'expansion de cette bulle conduit à la séparation du tissu avoisinant. La succession de milliers de bulles va permettre de réaliser un plan de clivage (horizontal, vertical ou oblique) à une profondeur souhaitée dans la cornée.

On entend, dans le cadre de la présente demande, par « point focal » une zone d'impact du faisceau laser correspondant à une situation où le spot du faisceau laser est formé dans un plan focal.

On entend, dans le cadre de la présente demande, par « surface de découpe » une surface comprenant un ensemble de points d'impact contigus formant un motif géométrique 2D ou 3D. A titre d'exemple, sur la figure 5 pour la première forme de trajectoire, la surface de découpe est formée par une spirale et par une surface cylindrique.

On entend, dans le cadre de la présente demande, par « plan de découpe » un plan (XY) comprenant un ensemble de points d'impact contigus formant un motif géométrique 2D.

On entend, dans le cadre de la présente demande, par « trajectoire prédéterminée tridimensionnelle » un ensemble de points formant une courbe établie par un logiciel. Cette courbe est ensuite décomposée en un ensemble de vecteurs contigus par le logiciel du système de déplacement du faisceau laser. Le système de déplacement du faisceau laser déplace le point focal du faisceau laser en suivant la trajectoire prédéterminée afin de former une série de points d'impact dans la cornée. La courbe est décrite dans la cornée en partant du point le plus profond dans la cornée vers le point le plus proche de la face avant de la cornée. En d'autres termes, le point de départ de la courbe commence sur un plan de découpe le plus profond vers le plan de découpe le plus superficiel de la cornée. La courbe matérialise des surfaces de découpe dont l'empilement forme un volume de découpe.

On entend, dans le cadre de la présente demande, par « élément de trajectoire » une partie d'une trajectoire 3D. Chaque trajectoire 3D peut être décomposée en une pluralité d'éléments de trajectoire. A titre d'exemple, la figure 5 présente trois exemples de trajectoires 3D correspondant à trois types de découpe. Dans le cas d'une découpe lamellaire antérieure type cylindrique, la trajectoire peut être formée d'une spirale et d'un cylindre. La spirale correspond à un élément de trajectoire formant ici une surface de découpe avec un ensemble de points contigus formant un motif de spirale. Le cylindre correspond à un autre élément de trajectoire décrivant une hélice ascendante dont le pas est ajustable. La surface de découpe et l'hélice forment un volume de découpe. Dans le cas d'une découpe top-hat, la trajectoire est formée d'une spirale, d'un cylindre, d'une spirale et d'un cylindre. Dans le cas d'une découpe Zig-Zag, la trajectoire est formée d'une spirale et de deux tronçons de cônes décrits par deux hélices à pas et à rayon variable placées en vis-à-vis. Un ensemble de paramètres qui sont la durée d'impulsion, l'énergie par impulsion, la cadence et la vitesse de balayage sont associés à chaque élément de trajectoire.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

De nombreux appareils de chirurgie de la cornée, du type LASIK sont basés sur un laser femtoseconde. En effet, la minimisation de la durée des impulsions est généralement préconisée pour la découpe des tissus transparents biologiques afin de minimiser le volume dans lequel est effectué le dépôt d'énergie et d'éviter un échauffement des tissus cornéens susceptibles d'entraîner leur endommagement irréversible. Il est difficile d'envisager la solution qui consisterait à augmenter simplement la durée d'impulsion afin de simplifier l'architecture de la source laser, et de ce fait augmenter sa compacité et réduire son poids. En effet, le seuil de photo-disruption dépend de l'intensité (W/cm²) ou de la densité d'énergie (W/cm3). Plus on augmente la durée d'impulsion plus il faut augmenter l'énergie pour atteindre ce seuil qui pourrait générer des effets thermiques.

Toutefois une constatation faisant partie de la présente invention est que l'ensemble des appareils de chirurgie ophtalmologique utilisant un laser femtoseconde sont basés sur des paramètres de découpe qui restent fixes au cours d'une même opération, c'est-à-dire pour l'ensemble de la trajectoire 3D du faisceau en n'importe quel point d'un volume de découpe dans la cornée. En particulier, ces appareils de l'art antérieur utilisent une durée d'impulsion constante au cours d'une opération de découpe. Or, on a constaté que dans le cas d'une impulsion femtoseconde, le dépôt d'énergie est généralement effectué sur un large volume en amont du point focal et autour du point focal. De ce fait, la densité d'énergie au point focal n'est pas forcément optimale, c'est-à-dire maximale au point focal.

Dans la présente demande, un des objectifs est de pouvoir déposer l'énergie au point focal, donc dans un petit volume afin de maximiser la densité d'énergie. Le fait d'optimiser la densité d'énergie permet de créer une bulle de cavitation avec un minimum d'énergie. L'optimisation ou la maximisation de la densité d'énergie est obtenue en variant dynamiquement la durée d'impulsion en fonction de la position du point focal dans la trajectoire 3 pendant la procédure de découpe dans la cornée.

La quantité d'énergie déposée ainsi que le volume dans lequel se fait le dépôt d'énergie varient en fonction de la durée d'impulsion afin de pouvoir agir sur le diamètre des bulles de cavitation pour garantir la qualité et la précision des découpes. De même, le fait d'agir sur le diamètre de bulle a une action sur la vitesse de découpe car la distance entre deux tirs successifs varie.

Une durée d'impulsion comprise entre 1 et 2 ps favorise un dépôt d'énergie localisé, formant des bulles de cavitation de faible diamètre. Ces petites bulles de quelques microns permettent de former une ligne de découpe plus lisse, et d'améliorer ainsi la qualité, la précision et la sélectivité de la découpe. A l'inverse, une durée d'impulsion de quelques centaines de femtoseconde conduit à un volume d'interaction plus important ce qui permet d'améliorer la vitesse de découpe au détriment de la qualité comme l'illustre la figure 4. En effet, la surface de découpe formée à partir des bulles de diamètre plus important est plus rugueuse. On utilisera donc une durée d'impulsion comprise entre 1 et 2 ps pour les éléments de trajectoires pour lesquels on veut favoriser la qualité de découpe et avoir une bonne sélectivité dans la direction de l'axe Z. A l'inverse on utilisera une durée d'impulsion de quelques centaines de femtosecondes pour les éléments de trajectoires pour lesquels on veut privilégier la vitesse de découpe.

La figure 6 montre une série d'images qui sont des vues latérales d'une zone irradiée par le dépôt de l'énergie au point focal. Les irradiations sont réalisées en faisant varier la durée d'impulsion entre 330 fs et 3 ps et en maintenant constants les autres paramètres du faisceau d'impulsion laser, c'est-à-dire la longueur d'onde à 1030 nm, la cadence à 500 kHz, la durée d'irradiation à 500 ms, l'énergie par impulsion à 2 µJ/s. Les images montrent qu'il y a une diminution de la dimension en volume lorsque la durée d'impulsion varie de 330 fs vers 1.8 ps et à partir de 1,8 ps, de manière surprenante, on note une inversion dans l'évolution de la dimension en volume lorsque la durée d'impulsion varie de 1,8 ps vers 3 ps. Les résultats d'irradiation montrent ici que la durée d'impulsion comprise entre 1,2 ps et 1,8 ps permet de former des bulles de cavitation de dimension en volume plus faible.

En plus de la variation dynamique de la durée d'impulsion, l'énergie de l'impulsion peut également être ajustée sur chaque élément de trajectoire de manière à se placer au seuil de photodisruption; lequel dépend de la profondeur du point focal et de la transparence de la cornée.

De même, la cadence du laser ainsi que la vitesse de balayage peuvent également ajustées de manière à former des bulles de cavitation contiguës induites par photodisruption.

La présente divulgation propose donc un appareil de chirurgie dédié en particulier à la découpe de la cornée basé sur l'utilisation d'un laser dont la durée d'impulsions est paramétrable sur chaque élément de trajectoire en fonction de la qualité et de la vitesse de découpe souhaitées.

L'appareil de la présente invention repose sur l'utilisation d'une unité de commande qui pilote la source laser et le système optique de déplacement du faisceau laser afin d'associer une durée d'impulsion optimale pour chaque élément de la trajectoire 3D du point focal dans la cornée pour favoriser soit une qualité de découpe par le confinement du dépôt d'énergie soit une vitesse de découpe au détriment de la qualité de découpe. L'unité de commande permet également d'associer une énergie, une cadence et une vitesse optimales pour chaque élément de trajectoire.

La figure 1 représente schématiquement un appareil de chirurgie ophtalmologique 10 selon un mode de réalisation. L'appareil est disposé vis-à-vis d'un oeil pour une intervention chirurgicale de découpe de la cornée. On a représenté ici schématiquement une vue en coupe d'une cornée 7 présentant une face externe et une face interne. On définit un axe optique de symétrie 8 passant par le centre de la cornée et étant perpendiculaire à la surface de la cornée. L'axe optique de symétrie 8 s'étend suivant une direction Z-Z orthogonale à un plan (XY). La cornée s'étend sensiblement dans le plan (XY). Le faisceau laser incident est focalisé à différentes profondeurs en volume dans la cornée selon une direction parallèle à l'axe Z avec un angle d'incidence normal.

L'appareil ophtalmologique 10 comprend une source laser picoseconde adaptée pour délivrer un faisceau d'impulsions laser, un système optique de focalisation 5 disposé sur le trajet optique du faisceau d'impulsions laser et adapté pour focaliser le faisceau d'impulsions en un point focal 15 dans l'épaisseur de la cornée 7 et un système optique de déplacement du faisceau d'impulsions laser 3, 4 pour déplacer le point focal suivant une trajectoire 3D prédéterminée.

La source laser est formée de deux parties distinctes, une première partie de la source laser 1.1 étant composées d'une cavité laser oscillatrice et d'un étireur et une seconde partie de la source laser 1.2 qui forme une tête laser amplificatrice, l'ensemble générant des impulsions ultra-courtes de l'ordre de quelques picosecondes à quelques centaines de femtosecondes. A titre d'exemple, pour réaliser une découpe dans un tissu cornéen, la source laser peut générer des impulsions des longueurs d'onde comprises entre 1030 nm et 1090 nm, d'énergie comprise entre 0,5 µJ et 20 µJ, avec une cadence comprise entre 1 Hz et 2 MHz, d'une durée d'impulsion comprise entre 350 fs et 3 ps. Une fibre optique souple 13 achemine le faisceau laser non-amplifié généré par la première partie de la source laser 1.1 vers la tête laser amplificatrice 1.2 qui comprend un amplificateur, un compresseur et un module acousto-optique, qui ne sont pas illustrés sur la figure 1.

De manière connue et dans le cadre de la présente invention, il est possible d'agir sur la durée d'impulsion en sortie de la source laser par une action sur le compresseur en allongeant la distance entre les deux réseaux diffractifs du compresseur avec une platine de translation motorisée. Cette action est pilotée par une unité de commande 2.

Comme indiqué ci-dessus, lors de l'impact du faisceau laser avec le milieu de la cornée, un plasma est généré par ionisation lorsque l'intensité du laser dépasse une valeur seuil, nommée seuil de claquage optique ou photodisruption. Une bulle de cavitation se forme alors, engendrant une disruption très localisée des tissus environnant. Ainsi, une découpe lamellaire du tissu cornéen consiste à réaliser une succession de petites bulles de cavitation adjacentes qui ont une dimension supérieure au diamètre du point d'impact. Ces bulles forment ensuite une ligne de découpe. La trajectoire du point focal du faisceau laser est située par exemple sur la surface d'un cylindre ou sur une hélicoïde présentant une symétrie axiale, par exemple de section elliptique ou circulaire et de dimension ou de diamètre déterminé. L'axe du cylindre peut est parallèle à l'axe Z-Z et est centré sur l'axe optique de symétrie de la cornée 8. L'axe du cylindre peut également être décentré par rapport à l'axe 8.

Le système optique de déplacement du faisceau laser est utilisé pour déplacer le point focal du faisceau laser dans la cornée suivant une trajectoire prédéterminée dans les trois directions X, Y et Z. Il comprend un premier scanner de balayage 3 permettant de déplacer le point focal suivant l'axe Z et un second scanner optique de balayage 4 permettant de déplacer le point focal suivant les deux axes X et Y dans un plan de focalisation (X, Y) correspondant au plan de découpe. Les deux scanners sont coordonnés et synchronisés.

Le premier scanner 3 comprend une pupille d'entrée pour recevoir le faisceau laser délivré par la tête laser amplificatrice 1.2 et une pupille de sortie de diamètre par exemple de 25,4 mm pour envoyer le faisceau laser vers le second scanner, et les vitesses de balayage selon l'axe Z sont comprises entre 10 mm/s et 400 mm/s. A titre d'exemple, le premier scanner est un télescope motorisé qui agit sur la collimation du faisceau laser et donc la position suivant l'axe Z-Z du point focal. Le premier scanner utilisé est par exemple un modèle Varioscan commercialisé par la société SCANLAB ou le modèle LS-scan Z commercialisé par la société LASEA.

Le second scanner 4, permettant la déflection optique du faisceau, comprend une pupille d'entrée pour recevoir le faisceau laser provenant du premier scanner et une pupille de sortie de diamètre de 20 mm pour envoyer le faisceau laser vers le système optique de focalisation 5. Il comprend par exemple deux miroirs optiques pivotant montés sur des axes pivotants, pilotés par des moteurs à induction, permettant de dévier le faisceau laser. La vitesse angulaire de chaque miroir correspond à une vitesse linéaire sur cible. Les vitesses de balayage obtenues sur cible selon l'axe X et l'axe Y sont comprises entre 1 mm/s et 5000 mm/s.

L'appareil de chirurgie ophtalmologique comprend une unité de commande 2 qui pilote de manière synchronisée la source laser et le système optique de déplacement du faisceau laser afin de varier dynamiquement un ensemble de paramètres de découpe associé à chaque impulsion, c'est-à-dire à chaque point focal ou point d'impact dans la cornée le long de la trajectoire 3D prédéterminée pour produire une bulle de cavitation avec une taille contrôlée.

L'unité de commande 2 permet de varier dynamiquement la durée d'impulsion, la vitesse de balayage, la cadence, l'énergie par impulsion en fonction de la position du point focal ou point d'impact dans la trajectoire 3D afin d'optimiser à la fois la qualité de la découpe et la vitesse de découpe.

L'unité de commande est connectée à la source laser et au système optique de déplacement du faisceau laser par des bus de communication permettant la transmission des consignes de commande aux différents éléments de l'appareil tels que :
- le signal d'activation de la source laser ;
- la durée d'impulsion du faisceau laser ;
- l'énergie par impulsion ;
- la cadence ;
- la vitesse de balayage ;
- la trajectoire 3D.

Selon un mode de réalisation de l'invention, l'unité de commande 2 est configurée pour piloter de manière synchronisée le premier scanner, le second scanner et la source laser via une interface logicielle pour faire varier la durée d'impulsion de la source laser en fonction de la position du point focal dans la trajectoire 3D prédéterminée. L'unité de commande 2 est configurée pour faire par exemple varier la durée d'impulsion entre 350 fs et 3 ps, de préférence entre 700 fs et 1,5 ps.

De même, l'unité de commande peut être configurée pour faire varier l'énergie par impulsion entre 0,5 µJ et 20 µJ pour chaque élément de trajectoire de manière à placer le point focal au seuil de photodisruption correspondant au seuil de claquage optique. En effet, le seuil de photodisruption étant dépendant de la profondeur d'impact dans la cornée et de la transparence de la cornée.

De même, l'unité de commande peut être configurée pour faire varier la vitesse Z entre 10 mm/s et 400 mm/s et la vitesse XY entre 1 mm/s et 5000 m/s et la cadence de balayage de manière à juxtaposer les bulles induites par les points focaux successifs.

Préalablement à la procédure de découpe, une trajectoire prédéterminée tridimensionnelle est chargée dans l'unité de commande. La trajectoire 3D est composée d'un ensemble de vecteurs extraits à partir d'une courbe. Ces vecteurs correspondent aux déplacements successifs du point focal dans la cornée. Chaque impulsion laser produit le long de ces vecteurs des bulles de cavitation contiguës. La distance entre les bulles successives dépend de la vitesse sur cible et de la cadence du laser. De préférence, il faut ajuster la vitesse par rapport à la cadence de manière à avoir des bulles juxtaposées.

De façon avantageuse, on peut décomposer chaque trajectoire en une pluralité d'éléments de trajectoire. Il est donc possible d'associer un ensemble de paramètres tels que la durée d'impulsion, l'énergie par impulsion, la cadence et la vitesse de balayage à chaque élément de trajectoire.

La figure 4 illustre schématiquement un exemple de trajectoire 3D 50 formée d'un premier élément de trajectoire sous forme d'une spirale 51 qui est un motif 2D et d'un second élément de trajectoire sous forme d'une hélice 52. Une telle trajectoire est particulièrement adaptée pour réaliser une découpe lamellaire antérieure dans la cornée.

L'unité de commande pilote les éléments de l'appareil afin d'associer une durée d'impulsion par exemple de 1,5 picoseconde pour tous les impacts laser délivrés sur le premier élément de la trajectoire pour privilégier la qualité de découpe et d'associer une durée d'impulsion par exemple de 500 fs pour tous les impacts laser délivrés sur le second élément de trajectoire pour privilégier la vitesse de découpe.

L'unité de commande pilote la source laser et le système optique de déplacement du faisceau de manière à former une première pluralité de bulles de cavitation 53 dont l'agencement forme une spirale. Une fois cette première pluralité de bulles de cavitation réalisées, l'unité de commande pilote la source laser et le système optique de déplacement du faisceau de manière à former une seconde pluralité de bulles de cavitation 53 dont l'agencement forme un motif hélicoïdal. Comme l'illustre l'exemple de la figure 4, les premières bulles formées ont une taille plus petite que celle des secondes bulles formées.

Le système optique de focalisation 5 est configuré de manière à focaliser le faisceau laser dans l'épaisseur de la cornée pour obtenir des points d'impact de taille constante à l'échelle de quelques microns sur un champ de travail de diamètre d'au moins 10 mm. L'axe optique de symétrie du système optique de focalisation est centré sur l'axe optique de symétrie 8 de la cornée. Selon une autre configuration, l'axe optique de symétrie du système optique de focalisation est décentré par rapport à l'axe optique de symétrie de la cornée.

Le système de focalisation 5 est constitué d'un assemblage de lentilles. Il convient de noter que les designs optiques ne sont pas définis ici, mais ils peuvent être donnés à titre indicatif comme un exemple de mise en oeuvre répondant pleinement au cahier des charges. Ainsi le nombre de lentilles, leurs caractéristiques et leur positionnement pourraient être différents tout en atteignant les caractéristiques techniques définies ci-dessus.

Selon une forme de réalisation de l'invention, le système de focalisation est une combinaison optique télécentrique. A titre d'exemple, la combinaison optique télécentrique utilisée peut présenter les caractéristiques suivantes :
- longueur d'onde : 1030 nm ;
- ouverture numérique : 0,15 ;
- distance de travail ou de focalisation : 50 mm ;
- pupille d'entrée : 15 mm ;
- taille de champ : 10 mm ;
- diamètre du point focal : 8,5 µm.

Selon une autre forme avantageuse de réalisation de l'invention, le système de focalisation est une combinaison optique télécentrique présentant les caractéristiques techniques suivantes :
- longueur d'onde : 1030 nm ;
- ouverture numérique : 0,22 ;
- distance de travail ou de focalisation : 30 mm ;
- pupille d'entrée : 14 mm ;
- taille de champ : 10 mm ;
- diamètre du point focal : inférieur à 6 µm, sur tout le champ de travail de 10 mm, à savoir en tout point de la cornée.

Cette seconde combinaison optique télécentrique permet d'offrir une ouverture numérique plus grande donc un point focal de plus faible diamètre, inférieur à 6 µm; elle permet donc une meilleure précision et une meilleure qualité de découpe que la première lentille pour laquelle le diamètre du spot laser est de l'ordre de 8,5 µm.

De manière avantageuse, les combinaisons optiques télécentriques de la présente invention sont configurées afin de combiner une forte ouverture numérique, de l'ordre de 0,22 pour la précision et la finesse de découpe et un grand champ de travail afin de couvrir toute la surface de la pupille fonctionnelle de l'œil, de l'ordre de 10 mm.

En outre, l'utilisation de cette combinaison spécifique combinée avec une durée d'impulsion optimisée permet de créer des petites bulles de quelques microns dans un grand champ de travail de l'ordre de 10mm, améliorant ainsi également la qualité de découpe avec des surfaces de découpe moins rugueuses.

De façon avantageuse, l'appareil comporte un dispositif interface d'aplanation 14 placé au contact de l'œil à traiter, qui permet de réduire l'angle d'incidence du faisceau laser sur la cornée 7. Ce dispositif interface comprend par exemple une lentille plan-concave dont la face disposée en regard de la cornée a un rayon de courbure supérieure ou égale au rayon de courbure moyen de la cornée. Selon une autre forme de réalisation, le dispositif interface comprend une lentille plan-plan. L'axe optique du dispositif interface d'aplanation est centré sur l'axe optique de symétrie 8 de la cornée. Le dispositif interface peut être fixé sur le système optique de focalisation de l'appareil. Dans ce cas, la hauteur du dispositif interface d'aplanation est précisément égale à la distance focale du système optique de focalisation.

De façon avantageuse et en référence aux figures 2A et 2B, l'appareil de chirurgie comprend des caméras 11, 12 permettant au chirurgien de visualiser sur un écran d'affichage 40 la cornée pendant l'opération et une caméra de centrage 9 dont le cône de vision est centré par rapport à l'axe optique de la cornée de manière à bien positionner l'appareil au-dessus de l'œil du patient.

Comme l'illustre la figure 2A, les deux caméras de vision 11, 12, dotées d'un anneau d'éclairage, positionnées de part et d'autre du système de focalisation 5 permettent de visualiser et illuminer l'œil pendant l'opération. Le diamètre de la zone d'image qui est d'environ 12 mm est supérieur à la zone de découpe où se déplace le spot laser piloté par le système optique de déplacement. Les deux caméras permettent d'observer la zone de découpe et de suivre le processus de découpe. Les deux caméras sont positionnées entre le système optique de focalisation et la cornée de sorte que le faisceau d'imagerie soit orienté d'un angle compris entre 45° et 47° par rapport à un axe optique de symétrie du système de focalisation. Ces valeurs permettent la visualisation de l'œil sur un champ de 10,5 mm tout en prenant en compte l'encombrement des optiques et mécaniques du dispositif, des caméras et du dispositif interface d'aplanation.

Comme l'illustre la figure 2B, la caméra de centrage 9 est une caméra amovible qui est agencée entre le système de focalisation et le tissu. Elle génère un cône de vision qui est renvoyé à 90° par un miroir 9.1 de manière à générer un cône de vision 9.2 centré sur l'axe optique 8 du système de focalisation, permettant ainsi de positionner l'appareil au-dessus de l'œil du patient. Avantageusement, la caméra est équipée d'un anneau d'éclairage générant un spot annulaire d'alignement permettant ainsi au patient de fixer un point lumineux centré sur l'axe optique de symétrie du système de focalisation.

Les trois caméras permettent ainsi de visualiser l'œil avant l'opération et un suivi pendant l'opération.

De manière avantageuse, l'appareil de chirurgie ophtalmologique permet de réaliser des découpes de qualité dans la cornée avec un laser à impulsions picosecondes. Un tel laser est compatible avec la transmission via une fibre optique, contrairement à un laser à impulsions femtosecondes qui délivre une intensité d'impulsions susceptibles d'endommager la fibre optique. Il existe des fibres optiques qui sont semi-rigides et qui peuvent transporter ces impulsions laser amplifiées, mais seulement dans une certaine limite d'énergie par impulsion. En outre, leur transmission optique n'est pas optimale, ce qui induit une perte de puissance significative en sortie de la fibre optique. De même, ces fibres optiques altèrent légèrement la polarisation initiale du faisceau laser. Selon une forme avantageuse de la présente invention, la source laser est formée de deux parties distinctes 1.1, et 1.2. La première partie 1.1 comprend une cavité laser oscillatrice et un étireur temporel, et la seconde partie, dite tête laser amplificatrice 1.2 comprend une cavité amplificatrice, un compresseur et un module acousto-optique qui ne sont pas illustrés sur les figures. Une fibre optique à maintien de polarisation 13 est interposée entre les deux parties distinctes pour transmettre le faisceau. Avec une telle architecture, l'impulsion laser issue de l'étireur temporel n'est pas encore amplifiée lors de son passage dans la fibre optique. Grâce à cette architecture spécifique, le système optique de déplacement du faisceau laser, le système optique de focalisation et la tête laser sont intégrés dans un module de découpe 20 qui est adapté pour être monté à l'extrémité d'un bras articulé auto-équilibré d'une amplitude de 1 mètre, permettant ainsi de dégager de l'espace autour du patient et de limiter l'encombrement au sol de l'appareil.

En référence à la figure 3, la présente invention concerne également un équipement de chirurgie ophtalmologique 100 dans lequel le module de découpe 20 est configuré pour être monté à une extrémité d'un bras articulé auto-équilibré 30 avec des moyens d'assistance et de blocage pneumatiques et l'autre extrémité du bras étant montée sur une baie électrotechnique mobile 60. Une partie des éléments de l'équipement sont logés dans la baie électrotechnique mobile 60, par exemple la première partie de la source laser 1.1 comprenant la cavité laser oscillatrice et l'étireur, l'écran d'affichage 40, l'unité de commande 2. Le bras permet de déplacer des charges de 0 à 35 kg avec un poids ressenti sensiblement nul. Le bras utilisé est par exemple le modèle Série 3 commercialisé par la société 3ARM. Des actionneurs positionnés sur le module de découpe permettent de déverrouiller le blocage pneumatique du bras afin de le déplacer selon les trois directions X, Y et Z. Le bras articulé auto-équilibré permet de positionner le module de découpe 20 au-dessus de l'œil du patient. L'ajustement du positionnement du module de découpe 20 est réalisé par le chirurgien aidé d'une caméra de centrage 9.

La présente invention permet de disposer d'un appareil de chirurgie ophtalmologique efficace et compact pour réaliser des découpes de grande qualité dans un tissu biologique oculaire, tel que la cornée ou le cristallin. Grâce à un système de focalisation avec une grande ouverture numérique, il est possible de réaliser des spots de taille micrométrique et homogène sur un champ de diamètre de 10 mm sur toute l'épaisseur de la couche de la cornée. En ajustant la durée d'impulsion du faisceau laser en fonction de la position du point focal dans la trajectoire pendant la découpe, il est également possible d'ajuster la taille des bulles de cavitation voulue. La combinaison d'une mise en forme à la fois spatiale, temporelle et dynamique de l'impulsion laser permet d'obtenir des découpes de qualité et de précision avec un laser picoseconde.

L'appareil de chirurgie ophtalmologique de la présente invention présente un coût de fabrication relativement faible, car il est équipé de composants simples et moins coûteux que les appareils actuels.

Il est plus compact et mobile grâce à une architecture spécifique dans laquelle le système de focalisation, le système de déplacement du faisceau et une partie des composants de la source laser sont intégrés dans un module de découpe compact et léger qui peut être monté à l'extrémité d'un bras articulé auto-équilibré.

L'utilisation d'une baie électrotechnique mobile permet d'intégrer le reste de l'équipement tel qu'une partie de la source laser, l'écran d'affichage pour pouvoir déplacer l'ensemble plus facilement.

L'utilisation d'une trajectoire 3D préétablie avec des paramètres de découpe optimisés et un système de déplacement du faisceau laser piloté permet une découpe semi-automatisée pour assurer la reproductibilité et la répétabilité.

Un des points intéressants de la technique mise au point dans la présente invention est de disposer d'une variation dynamique des paramètres de découpe tels que la durée d'impulsion, l'énergie par impulsion, la vitesse de déplacement du faisceau et la cadence en fonction de chaque élément de trajectoire, ce qui permet d'obtenir une découpe précise et fine, avec des surfaces de découpe de faible rugosité tout en garantissant une durée de découpe la plus courte possible. Un avantage technique qui en découlera directement est de pouvoir découper de fines couches et à amincir un greffon avec précision. Un autre avantage technique lié à cette trajectoire continue en dynamique, avec des impulsions de faible énergie est de mieux préserver le tissu cornéen grâce à des zones affectées thermiques très limitées.

Un autre avantage technique est de pouvoir proposer une découpe semi-automatique pendant toute l'opération. En revanche, le centrage de l'appareil, le déclenchement du faisceau laser et le choix de la trajectoire sont gérés par le chirurgien.

### Application industrielle

L'invention est particulièrement adaptée pour réaliser des opérations de découpe de cornée sur donneur et receveur en vue d'une greffe de cornée. Elle peut également être utilisée dans la préparation et l'amincissement de greffon. Elle peut être utilisée pour d'autres opérations de type LASIK, SMILE ou RELEX sans sortir du cadre de l'invention. Par exemple, l'invention trouve une application dans la chirurgie réfractive cornéenne, tel que le traitement des amétropies, notamment myopie, hypermétropie, astigmatisme. L'invention trouve également une application dans le traitement de la cataracte avec incision de la cornée. De manière générale, l'invention concerne toutes les opérations sur la cornée ou, par extension des capacités, le cristallin d'un oeil humain ou animal.

## Revendications

1. Appareil de chirurgie ophtalmologique (10) pour réaliser une découpe dans un tissu biologique oculaire (7), tel qu'une cornée ou un cristallin, comprenant :
- une source laser (1.1, 1.2) adaptée pour délivrer un faisceau d'impulsions laser,
- un système optique de focalisation (5) pour focaliser le faisceau d'impulsions laser en un point focal (15) dans le tissu biologique oculaire;
- un système optique de déplacement du faisceau d'impulsions laser (3, 4) configuré pour déplacer le point focal suivant une trajectoire tridimensionnelle prédéterminée;
- une unité de commande (2) configurée pour piloter la source laser (1.1, 1.2) et le système optique de déplacement du faisceau d'impulsions laser (3, 4) de sorte que les paramètres du faisceau d'impulsions laser et les paramètres du système optique de déplacement du faisceau d'impulsion laser soient ajustés en fonction de la position du point focal (15) dans la trajectoire pendant la découpe ;
- les paramètres étant la durée d'impulsion du faisceau laser, l'énergie par impulsion, la cadence d'impulsion du faisceau laser et la vitesse de balayage du faisceau laser ;
- ladite unité de commande (2) étant configurée pour piloter de manière synchronisée la source laser et le système optique de déplacement de sorte que la durée d'impulsion de la source laser varie en fonction de la position du point focal dans la trajectoire tridimensionnelle prédéterminée.

2. Appareil selon la revendication 1, dans lequel ladite unité de commande est apte à piloter la source laser de sorte que la durée d'impulsion du faisceau laser soit comprise entre 350 femtosecondes et 3 picosecondes, de préférence comprise entre 700 femtosecondes et 1,5 picosecondes.

3. Appareil selon la revendication 1 ou 2, dans lequel ladite unité de commande est apte à piloter la source laser de sorte que l'énergie par impulsion soit comprise entre 0,1 µJ et 20 µJ et la cadence comprise entre 50 kHz et 2 MHz, de préférence entre 50 kHz et 1 MHz.

4. Appareil selon l'une des revendications 1 à 3, dans lequel ladite unité de commande est apte à piloter le système de déplacement du faisceau d'impulsion laser de sorte que la vitesse de balayage soit comprise entre 0,1 m/s et 10 m/s.

5. Appareil selon l'une des revendications précédentes, dans lequel le système de déplacement du faisceau d'impulsions laser comporte un premier scanner (3) adapté pour recevoir le faisceau d'impulsions laser incident et configuré de manière à induire un déplacement du faisceau d'impulsions laser suivant un axe Z et un second scanner (4) adapté pour recevoir le faisceau d'impulsions laser incident et configuré de manière à induire un déplacement du faisceau dans un plan (XY).

6. Appareil selon l'une des revendications 1 à 5, dans lequel le système optique de focalisation (5) est disposé entre le système de déplacement optique (3, 4) et le tissu biologique (7) et configuré pour former dans le tissu biologique un point focal (15) de diamètre inférieur à 8,5 µm, de préférence inférieure à 6 µm sur un champ de diamètre compris entre 9 mm et 12 mm.

7. Appareil selon la revendication 6, dans lequel le système optique de focalisation (5) est constitué par une combinaison optique télécentrique comportant une ouverture numérique comprise entre 0,13 et 0,22, de préférence entre 0,20 et 0,22.

8. Appareil selon l'une des revendications précédentes, lequel comprenant en outre au moins une caméra (11, 12) configurée pour visualiser la zone de découpe.

9. Appareil selon la revendication 8, dans lequel ladite au moins une caméra (11, 12) est agencée entre le système optique de focalisation (5) et le tissu biologique (7) de sorte que le faisceau d'imagerie incident soit incliné d'un angle compris entre 30° et 50°, de préférence compris entre 45° et 47, par rapport à un axe optique de symétrie (8) du système optique de focalisation.

10. Appareil selon l'une des revendications précédentes, lequel comprenant en outre une caméra de centrage (9) configurée pour le centrage de l'axe optique de symétrie du système de focalisation par rapport au tissu biologique.

11. Appareil selon l'une des revendications précédentes, dans lequel la source laser (1.1, 1.2) émet des impulsions laser à une longueur d'onde comprise entre 1020 nm et 1600 nm, de préférence entre 1030 nm et 1090 nm.

12. Appareil selon l'une des revendications précédentes, lequel comprenant en outre un dispositif interface d'aplanation (14) comprenant une lame à faces planes et parallèles et/ou une lame plan-concave.

13. Appareil selon l'une des revendications précédentes, dans lequel ladite source laser étant formée de deux parties distinctes, la première partie (1.1) comportant une cavité laser oscillatrice et un étireur et la seconde partie (1.2) comportant une cavité laser amplificatrice, un compresseur et un module acousto-optique, ledit appareil comprend d'une part un module de découpe (20) intégrant le système optique de focalisation, le système optique de déplacement du faisceau d'impulsions laser et la seconde partie de la source laser, et d'autre part, une liaison à fibre optique (13) pour transmettre le faisceau laser généré par la première partie de la source laser (1.1) vers le module de découpe (20)

14. Équipement de chirurgie ophtalmologique (100) pour réaliser une découpe dans un tissu biologique oculaire (7), tel qu'une cornée ou un cristallin, comprenant un bras articulé auto-équilibré (30) suivant trois axes X, Y et Z, et un appareil de chirurgie ophtalmologique (10) selon la revendication 13, ledit bras comprenant une extrémité reliée à une baie électrotechnique mobile (60) et une extrémité adaptée pour être couplée au module de découpe (20).

## Patentansprüche

1. Ophthalmologische chirurgische Vorrichtung (10) zum Ausführen eines Schnitts in einem okularen biologischen Gewebe (7), wie z.B. einer Hornhaut oder einer Linse, umfassend:
- eine Laserquelle (1.1, 1.2), die dazu geeignet ist, einen Strahl von Laserimpulsen abzugeben,
- ein fokussierendes optisches System (5) zum Fokussieren des Laserimpulsstrahls auf einen Brennpunkt (15) im okularen biologischen Gewebe;
- ein optisches System zum Bewegen des Laserimpulsstrahls (3, 4), das so konfiguriert ist, dass es den Brennpunkt entlang einer vorbestimmten dreidimensionalen Bahn bewegt;
- eine Steuereinheit (2), die so konfiguriert ist, dass sie die Laserquelle (1.1, 1.2) und das optische System (3, 4) zur Bewegung des Laserimpulsstrahls so steuert, dass die Parameter des Laserimpulsstrahls und die Parameter des optischen Systems zur Bewegung des Laserimpulsstrahls in Abhängigkeit von der Position des Brennpunkts (15) in der Bahn während des Schnitts angepasst werden;
- wobei die Parameter die Impulsdauer des Laserstrahls, die Energie pro Impuls, die Impulsrate des Laserstrahls und die Abtastgeschwindigkeit des Laserstrahls sind;
- wobei die Steuereinheit (2) so konfiguriert ist, dass sie die Laserquelle und das optische Bewegungssystem synchron ansteuert, so dass sich die Impulsdauer der Laserquelle in Abhängigkeit von der Position des Brennpunkts in der vorbestimmten dreidimensionalen Bahn ändert.

2. Vorrichtung nach Anspruch 1, wobei die Steuereinheit geeignet ist, die Laserquelle so anzusteuern, dass die Impulsdauer des Laserstrahls zwischen 350 Femtosekunden und 3 Pikosekunden, vorzugsweise zwischen 700 Femtosekunden und 1,5 Pikosekunden, beträgt.

3. Vorrichtung nach Anspruch 1 oder 2, wobei die Steuereinheit die Laserquelle so ansteuern kann, dass die Energie pro Impuls zwischen 0,1 µJ und 20 µJ und die Rate zwischen 50 kHz und 2 MHz, vorzugsweise zwischen 50 kHz und 1 MHz, beträgt.

4. Vorrichtung nach einem der Ansprüche 1 bis 3, wobei die Steuereinheit dazu ausgelegt ist, das Bewegungssystem des Laserimpulsstrahls so zu steuern, dass die Abtastgeschwindigkeit zwischen 0,1 m/s und 10 m/s beträgt.

5. Vorrichtung nach einem der vorhergehenden Ansprüche, wobei das Laserimpulsstrahl-Bewegungssystem einen ersten Scanner (3) umfasst, der zum Empfangen des einfallenden Laserimpulsstrahls geeignet und so konfiguriert ist, dass er eine Bewegung des Laserimpulsstrahls entlang einer Z-Achse induziert, sowie einen zweiten Scanner (4), der zum Empfangen des einfallenden Laserimpulsstrahls geeignet und so konfiguriert ist, dass er eine Bewegung des Strahls in einer (XY)-Ebene induziert.

6. Vorrichtung nach einem der Ansprüche 1 bis 5, wobei das fokussierende optische System (5) zwischen dem optischen Bewegungssystem (3, 4) und dem biologischen Gewebe (7) angeordnet und so konfiguriert ist, dass es in dem biologischen Gewebe einen Brennpunkt (15) mit einem Durchmesser von weniger als 8,5 µm, vorzugsweise weniger als 6 µm, auf einem Feld mit einem Durchmesser zwischen 9 mm und 12 mm bildet.

7. Vorrichtung nach Anspruch 6, bei der das fokussierende optische System (5) von einer telezentrischen optischen Kombination mit einer numerischen Apertur zwischen 0,13 und 0,22, vorzugsweise zwischen 0,20 und 0,22, gebildet ist.

8. Vorrichtung nach einem der vorhergehenden Ansprüche, welche ferner wenigstens eine Kamera (11, 12) umfasst, die so konfiguriert ist, dass sie den Schneidbereich visualisiert.

9. Vorrichtung nach Anspruch 8, wobei die wenigstens eine Kamera (11, 12) zwischen dem fokussierenden optischen System (5) und dem biologischen Gewebe (7) so angeordnet ist, dass der einfallende Bildgebungsstrahl um einen Winkel zwischen 30° und 50°, vorzugsweise zwischen 45° und 47°, in Bezug auf eine optische Symmetrieachse (8) des fokussierenden optischen Systems geneigt ist.

10. Vorrichtung nach einem der vorhergehenden Ansprüche, welche ferner eine Zentrierkamera (9) umfasst, die zum Zentrieren der optischen Symmetrieachse des fokussierenden Systems in Bezug auf das biologische Gewebe konfiguriert ist.

11. Vorrichtung nach einem der vorhergehenden Ansprüche, wobei die Laserquelle (1.1, 1.2) Laserimpulse mit einer Wellenlänge zwischen 1020 nm und 1600 nm, vorzugsweise zwischen 1030 nm und 1090 nm, aussendet.

12. Vorrichtung nach einem der vorhergehenden Ansprüche, welche ferner eine Planarisierungs-Schnittstellenvorrichtung (14) umfasst, die eine Klinge mit ebenen und parallelen Flächen und/oder eine plankonkave Klinge umfasst.

13. Vorrichtung nach einem der vorhergehenden Ansprüche, wobei die Laserquelle aus zwei getrennten Teilen besteht, wobei der erste Teil (1.1) einen oszillierenden Laserresonator und einen Strecker umfasst und der zweite Teil (1.2) einen verstärkenden Laserresonator, einen Kompressor und ein akusto-optisches Modul umfasst, wobei die Vorrichtung einerseits ein Schneidmodul (20) umfasst, das das optische Fokussierungssystem, das optische System zur Bewegung des Laserimpulsstrahls und den zweiten Teil der Laserquelle integriert, und andererseits eine faseroptische Verbindung (13), um den vom ersten Teil der Laserquelle (1.1) erzeugten Laserstrahl zum Schneidmodul (20) zu übertragen.

14. Ophthalmologische chirurgische Ausrüstung (100) zum Ausführen eines Schnitts in einem okularen biologischen Gewebe (7), wie einer Hornhaut oder einer Linse, umfassend einen entlang dreier Achsen X, Y und Z selbstausgeglichenen Gelenkarm (30), und eine ophthalmologische chirurgische Vorrichtung (10) nach Anspruch 13, wobei der Arm ein Ende umfasst, das mit einem beweglichen elektrotechnischen Gestell (60) verbunden ist, sowie ein Ende, das dazu ausgelegt ist, mit dem Schneidmodul (20) gekoppelt zu werden.

## Claims

1. An ophthalmological surgical apparatus (10) for making a cut in an ocular biological tissue (7), such as a cornea or a crystalline lens, comprising:
- a laser source (1.1, 1.2) suitable for delivering a pulsed laser beam,
- a focusing optical system (5) for focusing the pulsed laser beam to a focal point (15) in the ocular biological tissue;
- an optical system (3, 4) for moving the pulsed laser beam, said system being configured to move the focal point along a predetermined three-dimensional path;
- a control unit (2) configured to control the laser source (1.1, 1.2) and the optical system (3, 4) for moving the pulsed laser beam so that the parameters of the pulsed laser beam and the parameters of the optical system for moving the pulsed laser beam are adjusted with respect to the position of the focal point (15) on the path during cutting;
- the parameters being the pulse duration of the laser beam, the energy per pulse, the pulse rate of the laser beam and the scan speed of the laser beam;
- said control unit (2) being configured to synchronously control the laser source and the moving optical system so that the pulse duration of the laser source varies with respect to the position of the focal point on the predetermined three-dimensional path.

2. The apparatus as claimed in claim 1, wherein said control unit is able to control the laser source so that the pulse duration of the laser beam is comprised between 350 femtoseconds and 3 picoseconds, and preferably comprised between 700 femtoseconds and 1.5 picoseconds.

3. The apparatus as claimed in claim 1 or 2, wherein said control unit is able to control the laser source so that the energy per pulse is comprised between 0.1 µJ and 20 µJ and the pulse rate comprised between 50 kHz and 2 MHz, and preferably between 50 kHz and 1 MHz.

4. The apparatus as claimed in one of claims 1 to 3, wherein said control unit is able to control the system for moving the pulsed laser beam so that the scan speed is comprised between 0.1 m/s and 10 m/s.

5. The apparatus as claimed in one of the preceding claims, wherein the system for moving the pulsed laser beam comprises a first scanner (3) suitable for receiving the incident pulsed laser beam and configured to induce a movement of the pulsed laser beam along an axis Z, and a second scanner (4) suitable for receiving the incident pulsed laser beam and configured to induce a movement of the beam in a plane (XY).

6. The apparatus as claimed in one of claims 1 to 5, wherein the focusing optical system (5) is placed between the moving optical system (3, 4) and the biological tissue (7) and configured to form, in the biological tissue, a focal point (15) of diameter smaller than 8.5 µm, and preferably smaller than 6 µm, over a field of diameter comprised between 9 mm and 12 mm.

7. The apparatus as claimed in claim 6, wherein the focusing optical system (5) consists of a telecentric optical combination having a numerical aperture comprised between 0.13 and 0.22, and preferably between 0.20 and 0.22.

8. The apparatus as claimed one of the preceding claims, which further comprises at least one camera (11, 12) configured to allow the cutting region to be viewed.

9. The apparatus as claimed in claim 8, wherein said at least one camera (11, 12) is arranged between the focusing optical system (5) and the biological tissue (7) so that the incident imaging beam is inclined by an angle comprised between 30° and 50°, and preferably comprised between 45° and 47°, with respect to an optical axis of symmetry (8) of the focusing optical system.

10. The apparatus as claimed in one of the preceding claims, which further comprises a centering camera (9) configured to center the optical axis of symmetry of the focusing system with respect to the biological tissue.

11. The apparatus as claimed in one of the preceding claims, wherein the laser source (1.1, 1.2) emits laser pulses at a wavelength comprised between 1020 nm and 1600 nm, and preferably between 1030 nm and 1090 nm.

12. The apparatus as claimed in one of the preceding claims, which further comprises a flattening interface device (14) comprising a plate with planar and parallel faces and/or a plano-concave plate.

13. The apparatus as claimed in one of the preceding claims, wherein said laser source being formed from two distinct parts, the first part (1.1) comprising an oscillating laser cavity and a stretcher and the second part (1.2) comprising an amplifying laser cavity, a compressor and an acousto-optical module, said apparatus comprises, on the one hand, a cutting module (20) incorporating the focusing optical system, the optical system for moving the pulsed laser beam and the second part of the laser source, and on the other hand, a fiber-optic link (13) for transmitting the laser beam generated by the first part of the laser source (1.1) to the cutting module (20).

14. An ophthalmological surgical equipment (100) for making a cut in an ocular biological tissue (7), such as a cornea or a crystalline lens, comprising a self-balancing arm (30) that is articulated about three axes X, Y and Z, and an ophthalmological surgical apparatus (10) as claimed in claim 13, said arm having one end connected to a mobile electrotechnical rack (60) and one end suitable for being coupled to the cutting module (20).
